# EUROPEAN PATENT APPLICATION

(11) **EP 4 806 399 A1**
(43) Date of publication of application: **16.09.2026**
(21) Application number: 25305342.5
(22) Date of filing: 13.03.2025
(51) Int. Cl.: A61F 2/958, G06T 7/62, A61B 6/50

(54) **BALLOON EXPANSION**

(71) Applicant: Koninklijke Philips N.V., 5656 AE Eindhoven (NL)
(72) Inventor: ALLAIRE, Stephane, 5656 Eindhoven (NL); AUVRAY, Vincent, 5656 Eindhoven (NL); OLIVAN BESCOS, Javier, 5656 Eindhoven (NL); POPOFF, Alexandre, 5656 Eindhoven (NL); LOBANTSEV, Artem, 5656 Eindhoven (NL)
(74) Representative: Philips Intellectual Property & Standards

(57) **Abstract**

The present invention relates to expanding a balloon. In order to provide further support during expansion of balloons, a device (10) for indicating balloon expansion eccentricity is provided that comprises a data input (12), a data processing arrangement (14) and an output interface (16). The data input is configured to receive 2D X-ray image data (18) of a region of interest comprising a portion of a vessel with an inserted guidewire and an inserted balloon being inflated. The data processing arrangement is configured to segment the balloon; to segment the guidewire; to analyze a spatial relation of the segmented balloon and the segmented guidewire; and to determine a measure of balloon expansion eccentricity (20) based on the analyzed spatial relation. The output interface is configured to output the measure of eccentricity.

## Description

### FIELD OF THE INVENTION

The present invention relates to expanding a balloon inside a vessel. The present invention relates in particular to a device for indicating balloon expansion, to a medical imaging system for support during balloon expansion and to a method for indicating balloon expansion.

### BACKGROUND OF THE INVENTION

An example of treatment is the use of inflatable balloons to expand the vessel after preparation of a calcified region of the vasculature. Balloon expansion can also be used during stent deployment and during post dilation. In the CathLab (catheterization laboratory), interventional radiologists (cardiologists, neurologists, etc.) may use live X-ray to check the optimal expansion of balloons. However, it has been shown that this requires experience and can be cumbersome.

### SUMMARY OF THE INVENTION

There may thus be a need for further support during expansion of balloons.

The object of the present invention is solved by the subject-matter of the independent claims; further embodiments are incorporated in the dependent claims. It should be noted that the following described aspects of the invention apply also for the device for indicating balloon expansion, for the medical imaging system for support during balloon expansion and for the method for indicating balloon expansion.

According to the present invention a device for indicating balloon expansion eccentricity is provided. The device comprises a data input, a data processing arrangement and an output interface. The data input is configured to receive 2D X-ray image data of a region of interest comprising a portion of a vessel with an inserted guidewire and an inserted balloon being inflated. The data processing arrangement is configured to segment the balloon. The data processing arrangement is also configured to segment the guidewire. The data processing arrangement is further configured to analyze a spatial relation of the segmented balloon and the segmented guidewire and to determine a measure of balloon expansion eccentricity based on the analyzed spatial relation. The output interface is configured to output the measure of eccentricity.

As an advantage, it is possible to identify situations, for instance during the necessary lesion preparation of calcified regions, in which it can occur that a balloon expansion is eccentric due to the calcium and where the guidewire is not in the center of the balloon.

The identification of the measure of eccentricity may help in avoiding suboptimal lesion preparation which could impact the minimum stent area (MSA) which is known to be important for long-term clinical outcome. The measure of eccentricity supports optimal balloon expansion that is also important during stent deployment and post-dilation optimization, as it directly impacts stent apposition.

According to an example, the data processing arrangement is configured to extract a centerline of the segmented balloon, and to analyze the spatial relation comprising to analyze a spatial relation of the extracted centerline and the segmented guidewire to determine the measure of eccentricity. As an effect, a robust detection is provided, since the centerline of the balloon has to be brought into relation with the guidewire.

According to an example, the data processing arrangement is configured to straighten the centerline, and to analyze the spatial relation of the extracted centerline and the segmented guidewire to determine the measure of eccentricity based on the straightened image data. The data processing arrangement is configured to provide the analysis of the spatial relation for an image section covering an axial portion of the balloon in a direction transverse to the centerline plus a determined tissue edge section. The data processing arrangement is configured to straighten the image data based on the centerline.

This provides a facilitated understanding of the eccentricity for the user.

In other words, instead of the complete image, only sections along the centerline are used for data processing, which provides a more efficient use of the data and the processing time.

Of advantage is that the image data processing is provided for those image parts that are of interest. As an effect, the eccentricity information can be provided in realtime, i.e. with a minimum delay.

In an example, for the straightening, the data processing arrangement is configured to identify image portions along the unstraightened centerline and to align them in their orientation to match with the straightened centerline.

This provides a better visibility of the eccentricity.

According to an example, the data processing arrangement is configured to move the image section along the guidewire.

As an advantage, a user is provided with the respective information in a focused manner.

According to an example, the 2D image data comprises a sequence of 2D X-ray images of the region of interest. The data processing arrangement is configured to repeat the segmenting steps and the analyzing steps, and to repeatedly update the measure of eccentricity.

This allows to provide a continuous stream of information that also allows to directly identify changes when navigating or when manipulating, i.e. operating the balloon.

In an option, the data processing arrangement is configured to provide summary measures.

According to an example, the device further comprises a display configured to present the measure of eccentricity. As an option, the display is also configured to display the 2D X-ray image data. This allows an immediate provision of the eccentricity.

According to an example, based on the measure of eccentricity, the data processing arrangement is configured to trigger a warning signal when a predetermined eccentricity threshold value is reached.

According to the present invention, also a medical imaging system for support during balloon expansion is provided. The system comprises an X-ray imaging arrangement and a device for indicating balloon expansion eccentricity according to one of the preceding examples. The X-ray imaging arrangement provides the 2D X-ray image data.

According to an example, the system further comprises a guidewire for insertion into a vascular structure and at least one inflatable balloon configured to be inserted into the vascular structure along the guidewire.

According to the present invention, also a method for indicating balloon expansion eccentricity is provided. The method comprises the following steps:
- Receiving 2D X-ray image data of a region of interest comprising a portion of a vessel with an inserted guidewire and an inserted balloon being inflated;
- Segmenting the balloon;
- Segmenting the guidewire;
- Analyzing a spatial relation of the segmented balloon and the segmented guidewire;
- Determining a measure of balloon expansion eccentricity based on the analyzed spatial relation; and
- Outputting the measure of eccentricity.
In an aspect, an eccentric balloon expansion indicator is provided.

According to an aspect, an automatic indicator of balloon expansion eccentricity is provided which can provide a quantitative measure. As an option, warnings are raised on the system, with potential informative visual displays to guide and support live assessment and treatment decisions.

An example for an area of application is in the field of CathLab procedures, e.g. percutaneous coronary interventions (PCI), e.g. in cathlabs. As an example, the cathlab is an examination room located in a hospital with equipment for diagnostic imaging to then visualize the arteries of the heart and the chambers of the heart and treat any stenosis or other abnormality found.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for indicating balloon expansion.
Fig. 2 shows an example of an X-ray image of a region of interest comprising a portion of a vessel with an inserted guidewire and an inserted balloon being inflated.
Fig. 3 shows an example of a medical imaging system for support during balloon expansion.
Fig. 4 shows basic steps of an example of a method for indicating balloon expansion.
Fig. 5 shows a further example of a workflow for indicating balloon expansion.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of", when preceding a list of elements, modify the entire list of elements and do not modify the individual elements of the list.

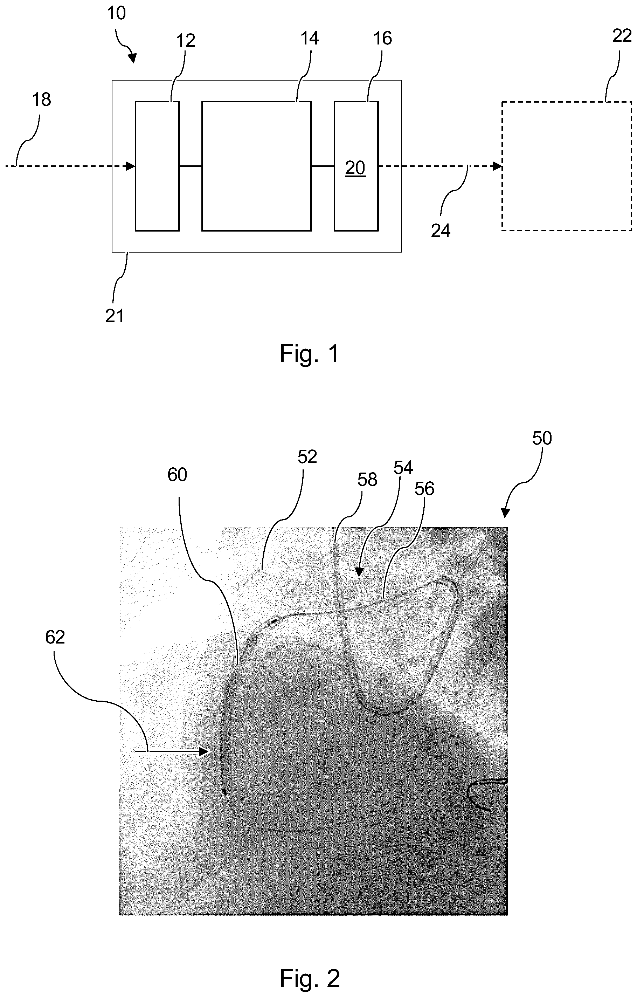

Fig. 1 schematically shows an example of a device 10 for indicating balloon expansion eccentricity. The device 10 comprises a data input 12, a data processing arrangement 14 and an output interface 16. The data input 12 is configured to receive 2D X-ray image data 18 of a region of interest comprising a portion of a vessel with an inserted guidewire and an inserted balloon being inflated. The data processing arrangement 14 is configured to segment the balloon, and to segment the guidewire. The data processing arrangement 14 is also configured to analyze a spatial relation of the segmented balloon and the segmented guidewire, and to determine a measure of balloon expansion eccentricity 20 based on the analyzed spatial relation. The output interface 16 is configured to output the measure of eccentricity 20.

A frame 21 indicates a common enclosing or supporting structure such as a housing. The data input 12, the data processing arrangement 14 and the output interface 16 can be arranged in an integrated manner. However, in another option, the data input 12, the data processing arrangement 14 and the output interface 16 are provided separately.

The term "to receive" relates to supplying image data, for example from an image source or from a database.

The term "2D X-ray image data" relates to 2D image data generated by X-ray radiation. For example, fluoroscopic images are provided.

The term "to segment" relates to identifying the balloon in its shape and distinguishing the image pixels representing the balloon from the surrounding image parts.

The term "to determine" relates to identifying e.g. by calculating the degree or value of eccentricity. The measure of eccentricity 20 can be given as percentage value or as actual distance value.

The term "eccentricity" relates to a deviation of the present situation from an ideal situation in which the balloon is inflated to the same degree around its longitudinal axis. The eccentricity is the shift of the cross-sectional form from the centerline axis.

The term "to output" relates to providing the measure of eccentricity 20 for further purposes, such as further computational steps or display steps.

The term "data input" relates to providing or supplying data for data processing steps. The data input 12 can also be referred to as image data input. The data input 12 can also be referred to as data supply, as image data supply, as image input, as input unit or simply as input. In an example, the data input 12 is data-connectable to an imaging source arrangement.

The term "data processing arrangement" relates to a processor or part of a processor arrangement that is provided to conduct the computing steps using the data supplied by the data input. The data processing arrangement 14 can also be referred to as data processor, as processor unit or as processor. In an example, the data processing arrangement 14 is data-connected to the data input 12 and the output interface 16.

The term "output interface" relates to an interface for providing the processed or computed data for further purposes. The output interface 16 can also be referred to as output or output unit. In an example, the output interface 16 is data-connectable to a display arrangement or display device. In another example, the output interface 16 is data-connected to a display. As an example, the signals by the controller can be provided by the output interface 16.

As an option, the device 10 further comprises a display 22 configured to present the measure of eccentricity. As an option, the display 22 is also configured to display the 2D X-ray image data.

An arrow 24 indicates a data-connection of the output interface 16 and the display 22.

The term "display" relates to a monitor or projector that is configured to show graphic information like X-ray images, Figures or instructions. The display 22 can be a wall-mounted arrangement or suspended from the ceiling. The display 22 can also be a head-worn display, for example for displaying of virtual reality or augmented reality.

Fig. 2 shows an example of an X-ray image 50 of a region of interest. The image 50 shows some anatomical structures like ribs 52 and vessels. A guidewire 54 is inserted in a portion of a vessel. The guidewire 54 may be running in loops, following the vascular paths. A part 56 is shown as wire only, and another part 58 is shown surrounded by a further structure. A further part shows an inflated balloon 60. An indicator 62 is provided that highlights a detected eccentricity.

In an option of the example of Fig. 1, the data processing arrangement 14 is configured to extract a centerline of the segmented balloon, and to analyze the spatial relation comprising to analyze a spatial relation of the extracted centerline and the segmented guidewire to determine the measure of eccentricity 20.

The term "extracting" relates to identifying and marking a virtual centerline of the balloon.

The centerline is determined as a line arranged in the middle of the respective width of the balloon.

In an option of the example of Fig. 1, the data processing arrangement 14 is configured to straighten the centerline, and to analyze the spatial relation of the extracted centerline and the segmented guidewire to determine the measure of eccentricity 20 based on the straightened image data. The data processing arrangement 14 is configured to provide the analysis of the spatial relation for an image section covering an axial portion of the balloon in a direction transverse to the centerline plus a determined tissue edge section. The data processing arrangement 14 is also configured to straighten the image data based on the centerline.

The term "straightened" relates to reducing its degree of curvature. In an example, the centerline is provided as a line with a minimal, i.e. small degree of curves. In another example, the centerline is straightened to have a linear extension (within the image plane).

This involves image resampling. The output un-warped image is handy for both simpler processing and helpful display for visual assessment.

The term "covering an axial portion" relates to a segment or part of the image that is extending to the left and right of the centerline to cover the whole expanding width of the balloon, but not its complete axial length. Thus, analysis is narrowed to a narrow band of the (unwarped) image astride the centerline.

In another option of the example of Fig. 1, the data processing arrangement 14 is configured to straighten the image data based on the centerline. For the straightening, the data processing arrangement 14 is configured to identify image portions along the unstraightened centerline and to align them in their orientation to match with the straightened centerline.

For the straightening, the data processing arrangement 14 is configured to identify image portions along the unstraightened centerline and to align them in their orientation to match with the straightened centerline.

In an example, the image portions remain un-warped.

In an option of the example of Fig. 1, the data processing arrangement 14 is configured to move the image section along the guidewire.

The term "moved along the guidewire" relates to a virtual application of the image section, e.g. like a window moved along the line.

In an option of the example of Fig. 1, the 2D image data 18 comprises a sequence of 2D X-ray images of the region of interest. The data processing arrangement 14 is configured to repeat the segmenting steps and the analyzing steps, to repeatedly update the measure of eccentricity 20, and to provide summary measures.

The term "sequence of 2D X-ray images" relates to a plurality of images, e.g. being acquired as successive images during a time span.

In a first option, the 2D X-ray image data 18 comprises fluoro images.

In a second option, the 2D X-ray image data 18 comprises X-ray cine image data.

An example of summary measures are e.g. minimum and maximum eccentricity.

In an option of the example of Fig. 1, for determining the measure of eccentricity, the data processing arrangement 14 is configured to take at least one of the following parameters into account: i) type of balloon being inflated, ii) type of inflation fluid; and iii) type of vessel in which the balloon is situated.

The term "type of balloon" relates to any feature characteristic for the balloon currently applied, which feature has an influence on the expandability when being inflated. The type can relate to a certain manufacturer, a certain form or shape, a certain material, a certain size and the like.

The term "type of inflation fluid" relates to the substance used for acting as pressure force on the inside of the balloon. The fluid can be a gaseous medium or a liquid medium.

The term "type of vessel" relates to any feature characteristic for the current vessel in which the balloon is inflated. The type of vessel can relate to the anatomic kind of vessel, a subject category comprising age, sex, size or weight of the subject, or vessel function such as cardiac vasculature.

As an example, the parameter is manually given or auto recognized or derived from automated procedure phasing combined with stent detection.

In an option of the example of Fig. 1, the data processing arrangement 14 is configured to highlight a current symmetry or eccentricity of the inflated balloon with respect to the guidewire through an overlay on the 2D X-ray image data 18.

The term "overlay" relates to an addition of a graphical element to the 2D X-ray image data.

In an example, a colorful overlay is provided.

As an example, a segmentation overlay is provided. As another example, a measurement overlay is provided. The display can be online and live during X-ray acquisitions.

In a further option of the example of Fig. 1, based on the measure of eccentricity 20, the data processing arrangement 14 is configured to trigger a warning signal when a predetermined eccentricity threshold value is reached.

The term "warning signal" relates to a signal to be received by the user to provide the information that the threshold value is reached. The warning signal can be a visual signal, an audible signal or a tactile signal or any combination thereof.

As an option, the device provides a Boolean flag that will either trigger a warning to the user, or else a positive control message.

In an option, the data processing arrangement 14 further comprises: i) a trained neural network that segments inflated balloons; and/or ii) a trained neural network that segments guidewires.

In an option, the data processing arrangement 14 further comprises an AI tool that is configured: i) to extract a centerline of elongated structures and to apply the extraction of the centerline to the segmented balloon; and/or ii) to analyze a shape and position of each segmented balloon with respect to the overlapping segmented guidewire and to output a measure of eccentricity.

In an example, the AI tool is configured to trigger warnings to the user based on the measure of eccentricity.

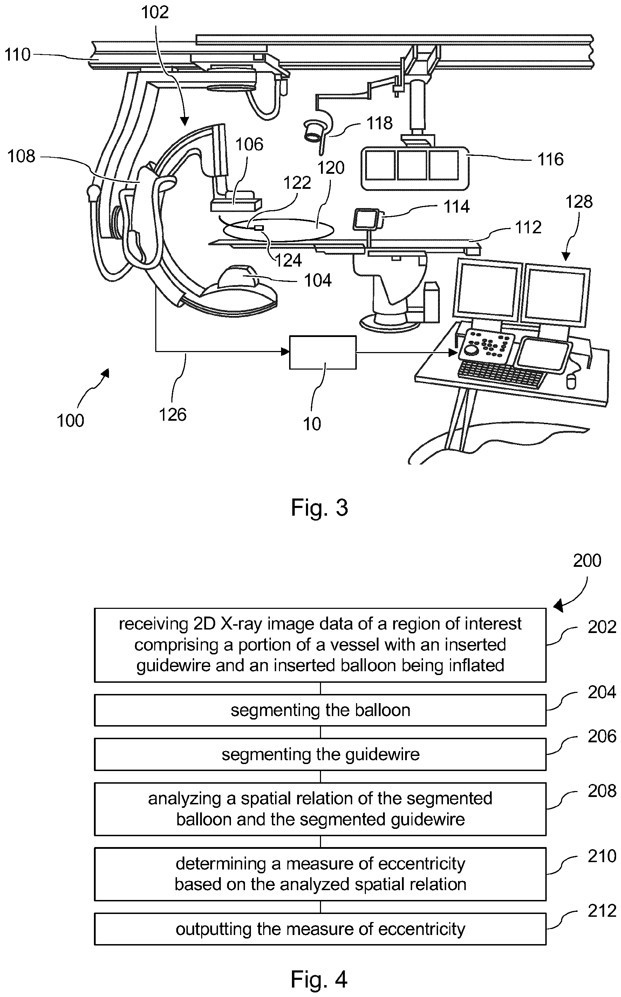

Fig. 3 shows an example of a medical imaging system 100 for support during balloon expansion. The medical imaging system 100 comprises an X-ray imaging arrangement 102 and an example of the device 10 for indicating balloon expansion eccentricity according to one of the preceding and following examples. The X-ray imaging arrangement 102 provides the 2D X-ray image data 18.

The system 100 provides a quantitative measure or a percentage of eccentricity.

In Fig. 3, the X-ray imaging arrangement 102 is shown comprising an X-ray source 104 and an X-ray detector 106 mounted to opposing ends of a movable C-arm arrangement 108 that is suspended from a ceiling rail system 110. Further, a subject support 112 is shown with a bedside controller interface 114. Still further, a display arrangement 116 and some lighting equipment 118 can be provided. A subject 120, e.g. a patient, is arranged on the subject support 112.

In Fig. 3, as an option, the system 100 further comprises a guidewire 122 for insertion into a vascular structure of the subject. The system 100 also comprises at least one inflatable balloon 124 configured to be inserted into the vascular structure along the guidewire 122.

In Fig. 3, the guidewire 122 is inserted and the inflatable balloon 124 is inserted along the guidewire 122. Further components like an operating handle of the guidewire or a control interface for the balloon are provided, but not shown.

The X-ray imaging arrangement 102 is data-connected to the device 10 for indicating balloon expansion by a data-connection, which can be wire-bound or wireless.

As an option, Fig. 3 shows a console 128 in the right foreground that may comprise interface components like a display, keyboard, mouse, trackpad, control knobs and the like. The console is provided for operating and controlling the various equipment.

Fig. 4 shows basic steps of an example of a method 200 for indicating balloon expansion eccentricity. The method 200 comprises the following steps:
- In a first step 202, 2D X-ray image data of a region of interest is received, the data comprising a portion of a vessel with an inserted guidewire and an inserted balloon being inflated.
- In a second step 204, the balloon is segmented.
- In a third step 206, the guidewire is segmented.
- In a fourth step 208, a spatial relation of the segmented balloon and the segmented guidewire is analyzed.
- In a fifth step 210, a measure of balloon expansion eccentricity is determined based on the analyzed spatial relation.
- In a sixth step 212, the measure of eccentricity is output.

In an option, the segmenting of the balloon and the segmenting of the guidewire are provided in a different order or at the same time.

In an example of the method, it is further provided the step of extracting a centerline of the segmented balloon. The analyzing of the spatial relation comprises analyzing a spatial relation of the extracted centerline and the segmented guidewire to determine the measure of eccentricity.

In an example of the method, it is further provided that the centerline is straightened. The analyzing of the spatial relation of the extracted centerline and the segmented guidewire to determine the measure of eccentricity are based on the straightened image data.

In an example of the method, it is further provided that the image data is straightened based on the centerline. For straightening, image portions along the unstraightened centerline are identified and aligned in their orientation to match with the straightened centerline.

In an example of the method, it is further provided that the analyzing of the spatial relation is provided for an image section covering an axial portion of the balloon in a direction transverse to the centerline plus a determined tissue edge section.

In an example of the method, it is further provided that the image section is moved along the guidewire.

In an example of the method, it is further provided that the 2D image data comprises a sequence of 2D X-ray images of the region of interest; the segmenting steps and the analyzing steps are repeated. The measure of eccentricity is updated repeatedly.

In an example of the method, it is further provided that, for determining the measure of eccentricity, at least one of the following parameters is accounted for: i) type of balloon being inflated; ii) type of inflation fluid; and iii) type of vessel in which the balloon is situated.

In an example of the method, it is further provided that a current symmetry or eccentricity of the inflated balloon with respect to the guidewire is highlighted through a colorful overlay on the X-ray image data.

In an example of the method, it is further provided that, based on the measure of eccentricity, a warning signal is triggered when a predetermined eccentricity threshold value is reached.

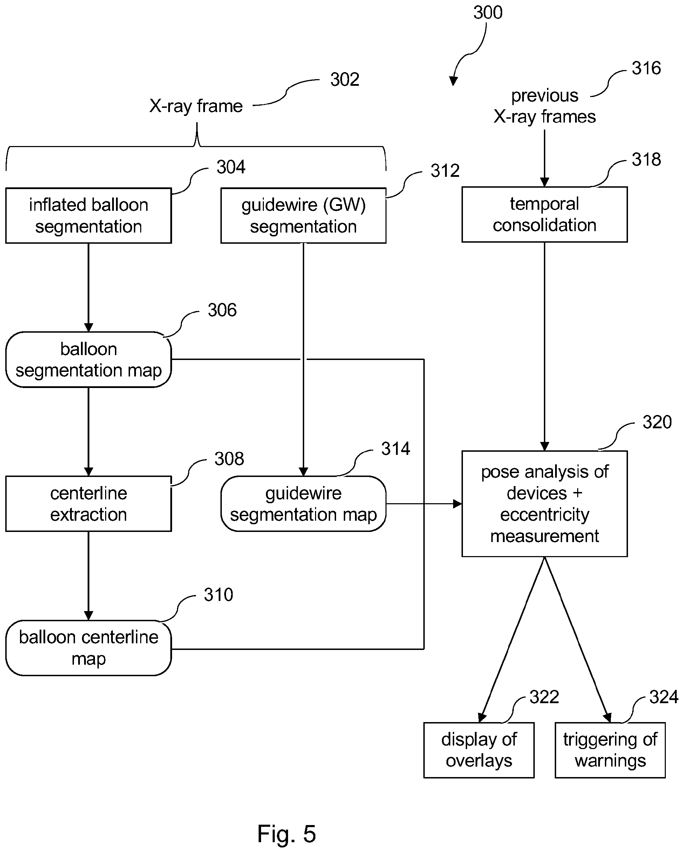

Fig. 5 shows a further example of a workflow 300 for indicating balloon expansion. An X-ray frame 302 is provided as a starting point. The image, i.e. the X-ray frame 302, is then subject to an inflated balloon segmentation 304. As a result, a balloon segmentation map 306 is provided. This is then subject to a centerline extraction 308, resulting in a balloon centerline map 310. Further, the image, i.e. the X-ray frame 302, is also subject to a guidewire segmentation 312. As a result, a guidewire segmentation map 314 is provided. As a third branch, previous X-ray frames 316 are provided to a temporal consolidation 318. The result of the temporal consolidation 318 is subject to a pose analysis of devices and eccentricity measurement 320. This process is also supplied with the balloon segmentation map 306, the balloon centerline map 310 and the guidewire segmentation map 314. As an output of the pose analysis of devices and eccentricity measurement 320, a display of overlays 322 is provided. Another option is the triggering of warnings 324 as an output. In a variation, overlays and warnings are provided at the same time.

In an example, a computer program is provided comprising instructions which, when the program is executed by a computer, cause the computer to carry out the method of the preceding examples.

In an example, a computer program or program element for controlling an apparatus according to one of the examples above is provided, which program or program element, when being executed by a processing unit, is adapted to perform the method steps of one of the method examples above.

In an example, a computer readable medium having stored the computer program of the preceding example is provided.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for indicating balloon expansion eccentricity, comprising:
- a data input (12);
- a data processing arrangement (14); and
- an output interface (16);
wherein the data input is configured to receive 2D X-ray image data (18) of a region of interest comprising a portion of a vessel with an inserted guidewire and an inserted balloon being inflated;
wherein the data processing arrangement is configured to segment the balloon; to segment the guidewire; to analyze a spatial relation of the segmented balloon and the segmented guidewire; and to determine a measure of balloon expansion eccentricity (20) based on the analyzed spatial relation; and
wherein the output interface is configured to output the measure of eccentricity.

2. Device according to claim 1, wherein the data processing arrangement is configured to extract a centerline of the segmented balloon; and to analyze the spatial relation comprising to analyze a spatial relation of the extracted centerline and the segmented guidewire to determine the measure of eccentricity.

3. Device according to claim 1 or 2, wherein the data processing arrangement is configured to straighten the centerline; and to analyze the spatial relation of the extracted centerline and the segmented guidewire to determine the measure of eccentricity based on the straightened image data;
wherein the data processing arrangement is configured to provide the analysis of the spatial relation for an image section covering an axial portion of the balloon in a direction transverse to the centerline plus a determined tissue edge section; and
wherein the data processing arrangement is configured to straighten the image data based on the centerline.

4. Device according to claim 3, wherein the data processing arrangement is configured to move the image section along the guidewire.

5. Device according to one of the preceding claims, wherein the 2D image data comprises a sequence of 2D X-ray images of the region of interest; and
wherein the data processing arrangement is configured to repeat the segmenting steps and the analyzing steps; to repeatedly update the measure of eccentricity; and to provide summary measures.

6. Device according to one of the preceding claims, wherein, for determining the measure of eccentricity, the data processing arrangement is configured to take at least one of the following parameters into account:
i) type of balloon being inflated,
ii) type of inflation fluid; and
iii) type of vessel in which the balloon is situated.

7. Device according to one of the preceding claims, further comprising a display (22) configured to present the measure of eccentricity; and
wherein the display is also configured to display the 2D X-ray image data.

8. Device according to claim 7, wherein the data processing arrangement is configured to highlight a current symmetry or eccentricity of the inflated balloon with respect to the guidewire through an overlay on the 2D X-ray image data.

9. Device according to one of the preceding claims, wherein, based on the measure of eccentricity, the data processing arrangement is configured to trigger a warning signal when a predetermined eccentricity threshold value is reached.

10. A medical imaging system (100) for support during balloon expansion, the system comprising:
- an X-ray imaging arrangement (102); and
- a device (10) for indicating balloon expansion eccentricity according to one of the preceding claims;
wherein the X-ray imaging arrangement provides the 2D X-ray image data.

11. Imaging system according to claim 10, further comprising:
- a guidewire (122) for insertion into a vascular structure; and
- at least one inflatable balloon (124) configured to be inserted into the vascular structure along the guidewire.

12. A method (200) for indicating balloon expansion eccentricity, the method comprising the following steps:
- receiving (202) 2D X-ray image data of a region of interest comprising a portion of a vessel with an inserted guidewire and an inserted balloon being inflated; and
- segmenting (204) the balloon;
- segmenting (206) the guidewire;
- analyzing (208) a spatial relation of the segmented balloon and the segmented guidewire;
- determining (210) a measure of balloon expansion eccentricity based on the analyzed spatial relation; and
- outputting (212) the measure of eccentricity.

13. A computer program comprising instructions which, when the program is executed by a computer, cause the computer to carry out the method of claim 12.
